# EUROPEAN PATENT APPLICATION

(11) **EP 2 572 740 A1**
(43) Date of publication of application: **27.03.2013**
(21) Application number: 11007643.7
(22) Date of filing: 20.09.2011
(51) Int. Cl.: A61M 5/20, A61B 5/145, A61M 5/172

(54) **Drug injection devices and systems with an analyte measurement module**

(71) Applicant: Roche Diagnostics GmbH, 68305 Mannheim (DE); F.Hoffmann-La Roche AG, 4070 Basel (CH)
(72) Inventor: Arnold, Ofer, Ha´movil 17920 (IL); Shergold, Oliver, 3065 Bolligen (CH); Yodfat, Ofer, Modi´in 71725 (IL)

(57) **Abstract**

Disclosed is a drug injection device, comprising a drug delivery module and an analyte measurement module, such as a blood glucose meter. The device can be provided with I/O means and can be further adapted to communicate with other networked medical or IT devices. Various options are disclosed: different energy sources, a code reader adapted to identify a previously coded drug cartridge during or after insertion, a plurality of different drug cartridges, a sprinkler needle to optimize injections, an adaptor for connection to an infusion set or to a micro pump, a cap covering the needle and containing lancing elements as well as blood test strips, an accessory base station for energy transfer and/or data exchange. Associated systems and methods are disclosed.

## Description

Examples of drug insulin injection devices are described. Specifically, there are described insulin pen devices integrated with a blood glucose meter adapted to diabetes management. Associated systems and methods are described.

### Background

A standard or conventional insulin pen is used to inject insulin for the treatment of diabetes. It is composed of an insulin cartridge (integrated or bought separately), a spring drive to force insulin from the cartridge and a dial to set the dose delivered. The pen is used with disposable needles. A durable pen uses a replaceable insulin cartridge. When the insulin cartridge is empty, the empty cartridge is disposed of and a new one is inserted in the pen. A prefilled pen is entirely disposable. The pen comes pre-filled with insulin, and when the insulin cartridge or reservoir is empty, the entire unit is discarded.

US Patent 6454746 entitled "Medication delivery apparatus" discloses a medication delivery apparatus including a housing, a container of medication mounted to the housing, an outlet such as an injection needle, and a drive assembly adapted to force medication from the container and through the outlet upon movement of an actuator. The housing periphery includes a projecting abutment for digit engagement which is ergonomically designed for abutting contact by a first digit of a hand of a user when the housing is grasped within the user's hand such that a second digit of the hand of the user may operate the actuator. When the actuator is moved axially, this abutting contact of the first digit with the abutment permits application of an axial force by the first digit on the abutment in a direction generally opposite to an axial force applied to move the actuator. As this user applied force on the abutment reduces the amount of frictional force required to be applied by the user on the apparatus housing to counteract the force applied to the actuator during medication delivery, the apparatus housing may be gripped less tightly during use. The housing also is provided with a gripping layer on its periphery formed of a soft touch material.

This solution presents drawbacks and limitations for diabetes management. For example, the patient must use other devices in conjunction to the presented pen device, which remains "passive" and does not offer more advanced features that contribute to better management of the user's diabetes. For example, with a conventional insulin pen a patient is not able to mix two different insulin (for example rapid and long acting) in the same pen. There are many other disadvantages and limitations with standard insulin pens.

There is a need for improved insulin pen delivery devices, systems and associated methods.

### Summary

There are disclosed devices, systems and methods of administrating drug to a patient, in particular insulin.

The medical product described in the present disclosure is provided with a BGM (Blood Glucose Meter, or Measurement or Monitoring system). Said insulin pen comprising a BGM may be hereafter referred to as the "*pen-creas*" insulin pen, or "intelligent" insulin pen, or as the pen according to embodiments of the present disclosure, as opposed to a standard or conventional or classic insulin pen.

A Blood Glucose Meter (or Measurement, or Monitoring) is used for testing the concentration of glucose in the blood to assess the glycemic state of a patient. A blood glucose test is performed by piercing the skin (typically on the finger) to draw blood, then applying the blood to a chemically active disposable 'test-strip'. Persons with Type 2 diabetes test at least once per day. Persons with Type 1 usually test their blood sugar more often (3 to 10 times per day), both to assess the effectiveness of their prior insulin dose and to help determine their next insulin dose. The term "Monitoring" often refers to regular (as opposed to punctual) measurements. Both embodiments are covered in the present disclosure.

In a particular embodiment, the drug injection device presents the shape of a pen (elongated device, i.e. approximately cylindrical with a ratio length/diameter superior to 1). But in other embodiments, the shape can be very different (front view or section as a square, star, oval, triangle, rectangular, trapezoidal, polygon). The housing containing the different components can be rigid or flexible (adapted to deformations or to conform to the body of the patient). Noticeably the device can be "distributed" over several (communicating) parts and disposed on or in or near the body of the patient (system of devices).

Effects or advantages or objectives of the present disclosure relate to an increased comfort of use, a better glycemic control, an optimized diabetes management, an enhanced portability, an increased discreteness, a new user interaction model, and new or complimentary administration schemes.

The disclosure enables diabetes management system in one device. The advising system is based on data collected from multiple sources (internal and external inputs). The operation can be fully automatic and electronic, including alerts, alarms, and reminders. The presented solution is compatible with off-the-shelf (all insulin vendors) and proprietary (multi-volume) cartridges.

New or complementarity treatments in diabetes management are enabled. The device indeed combines advantages and functions of different devices. For example, the presented insulin pen complements or replaces a traditional or micro or patch insulin pump (for example, during the day when the patient is awakened, with an adapted basal replacement if required), it complements or replaces a lancing device used to perform blood samples withdrawals, and it complements or replaces a stand-alone blood glucose meter.

The convenience of the use of a device in a pen shape enables increased discreteness (due to the shape factor which is similar to a widely distributed object, the attention of third parties is not drawn to the pen).

A way of using the device and the associated diabetes management can also be deeply modified. For example, it becomes possible to compensate for a basal delivery during a disconnection of an insulin pump by applying frequent injections. During sport or exercising, it is possible to administer a different (for example, a degraded) basal rate, in absence of a pump.

The portability of the device opens many new possibilities. For example, it enables a better calibration of a Continuous Monitoring sensor or device, if in use. Therefore, it enables a diabetic to make more frequent measurements in order to reach a better glycemic control.

As an intelligent and connected device, the insulin pen can cooperate with other networked medical devices and optimize the management of diabetes. For example, the bolus calculator of the "intelligent" (connected) insulin pen can manage downstream boluses versus upstream boluses (subtracting, fractioning, etc). In other words, the management of boli is improved or optimized, because of the cooperation with networked devices in an integrated system.

There is disclosed an injection device for delivering a fluid, the injection device comprising a support for receiving a reservoir or a cartridge, a drive means for expelling the fluid from the reservoir or cartridge, an injection means fluidically coupled to the reservoir characterized in that the injection device comprises an analyte measurement module.

As a first development, the delivery of the fluid corresponds to a prick, or is configured to oscillate back and forth along a longitudinal axis as the liquid is dispensed from the fluid injection means.

As another development, the analyte measurement module is part of a housing of the injection device.

As another development, the analyte measurement module is a blood glucose meter and the fluid is insulin.

As another development, the device comprises a user interface including data inputting and outputting means, such as a microphone, a speaker, a touch-sensitive screen, a slidable cursor, a physical button, a camera, or a combination thereof.

As another development, the device further comprises communication means, adapted to communicate with other networked devices, such as an insulin pump, an insulin pen, a continuous monitoring sensor, a personal computer, a mobile phone, a tablet, a television or a combination thereof.

As another development, the energy source is one of a dynamo, or a spring, or a rechargeable battery, or a solar cell or a combination thereof.

As another development, the device comprises a code reader adapted to identify a previously coded drug cartridge.

As another development, the device comprises a plurality of drug cartridges, prefilled or not, and/or drug reservoirs, such as rapid and long acting insulin.

As another development, the device comprises an injection needle.

As another development, the needle is a sprinkler needle comprising a plurality of holes for optimizing insulin depots during injection.

As another development, there is disclosed an adapter to said needle, said adapter being connectable via a tubing to an infusion set or a patch-pump inlet.

As another development, the device comprises a cap covering said needle, containing lancing elements and one or more blood test strips compatible with the blood glucose meter.

As another development, the device is adapted to be received in a base station, wherein said base station is suitable for energy transfer and/or data exchange.

There is disclosed a medical system comprising a pen-shaped medical device and comprising computing means adapted to implement one or more functions of a bolus calculator, the implementation of said computing means being local, for example in a processor, or said computing means being accessed remotely via a network.

There is also disclosed a medical system comprising a plurality of cooperating pen-shaped medical devices and/or with a standard pen mounted with a communication cap comprising a sensor and communications means.

There is also disclosed a cap for an insulin pen comprising test strips, desiccant elements, and lancing elements.

The present disclosure describes several embodiments of a drug pen devices, and associated systems and methods.

### Brief description of the figures

Figure 1 shows a global environment of the insulin pen or injection device interacting with other networked medical devices and IT devices (information technology), for example, a personal computer, a mobile phone, Tablet PC, and another insulin pen;
Figure 2 shows an insulin pen (injection device) integrated with a BGM;
Figure 3 shows an injection device (insulin pen) provided with a communication channel, for example a USB port;
Figure 4 illustrates an exemplary workflow of the bolus calculator;
Figure 5 shows insulin cartridges of different sizes that can be used in an embodiment of the disclosed device;
Figure 6 shows an insulin pen (injection device) according to the disclosures that is provided with a bar code reader and a communication channel;
Figures 7A to 7B show another exemplary insulin pen (injection device) according to the disclosure comprising a socket for receiving an insulin cartridge;
Figure 8 shows an embodiment of insulin pen (injection device) comprising two reservoirs, for example, comprising rapid and long acting insulin;
Figure 9A to 9D show an embodiment of an adapter and method for refilling an insulin pen (injection device);
Figure 10 shows an exemplary user interface of an insulin pen (injection device), for example, comprising a slidable element and/or a touchscreen and/or a microphone and/or physical input button and/or a combination thereof;
Figure 11A to 11G illustrates the actuation of a BGM of an embodiment of the disclosed device;
Figure 12 shows a particular embodiment where an insulin pen (injection device) is provided with a sprinkler needle instead of a standard needle;
Figure 13 shows an embodiment of an insulin pen (injection device) provided with an adapter for a connection to standard infusion set through tubing;
Figure 14 shows an example of an insulin pen (injection device) provided with an adapter for a direct connection to standard infusion set;
Figures 15 and 16 show an indicator for a disclosed insulin pen (injection device);
Figure 17 shows an embodiment of a cap for an insulin pen (injection device) comprising lancing elements held within the cap of the insulin pen;
Figure 18 shows a base station for receiving the insulin pen (injection device) and having dual functions in this particular embodiment (recharge battery and data transfer);
Figure 19A shows an exemplary adapter for enabling a communication channel to a standard insulin pen;
Figure 19B shows an insulin pen (injection device) according to the present disclosure communicating with a standard or conventional insulin pen;
Figure 20 shows a motorized insulin pen (injection device) according to the disclosure;
Figure 21A to 21C show the cap comprising lancing elements and test strips, as well as a strip compartment cover with desiccant;
Figure 22 shows steps of an example of the use of the injection device with the cap comprising lancing elements and test strips;
Figure 23 shows different adaptors to accommodate different insulin cartridges.

### Detailed description

Values such as amounts of insulin displayed that are discussed and/or illustrated in the figures of the present description are only examples. These values may cause injuries or death if inappropriately applied.

In one embodiment, the injection device is provided with a single housing that accommodates:
- a drug delivery module, including (at least) a motor, gears, a plunger and a cartridge/reservoir;
- an analyte monitoring module including (at least) a BGM;
- a processor to manage the delivery of the drug, analyte sensing/monitoring, and interface (I/O) with the user;

The single housing optionally includes input means and output means. In one embodiment, the input means and output means are both implemented, at least in part, in a single touch-sensitive screen. Thus, the operation of the injection device (both drug delivery and analyte sensing/monitoring) is being done digitally at the injection device housing, being controlled by a single processor. In some embodiments, the injection device includes a bolus calculator feature (operated in conjunction with the processor). In some embodiments, the drug/therapeutic fluid comprises insulin and the analyte comprises glucose (i.e., body glucose).

An example of usage of such an injection device may include the following steps:
- the user inserts a blog glucose (BG) strip to a dedicated port located at the injection device housing;
- the processor (accommodated within the housing) receives the BG reading/value from the strip;
- the user inputs an estimation of carbs to be ingested (for example via a touch-sensitive screen located at the housing);
- the processor (via bolus calculator feature) determines the recommended bolus amount to be delivered to the user, based on at least the BG reading and the carbs estimation. In some embodiments, other parameters (either input by the user or retrieved from a memory accommodated within the housing or elsewhere through, for example, a wireless connection) are taken into account in this bolus amount calculation. Additional parameters may include one or more of Carbs-to-Insulin Ration ("CIR"; which may be also be referred to as and insulin-to-carbs ration, or "ITC"), Insulin Sensitivity Factor ("ISF"; which may be also referred to simply as "IS"), Target Blood Glucose ("TBG"), Bolus-on-Board ("BOB"; may be also referred-to as "Insulin-On-Board" and "Residual Insulin"), and the like;
- the recommended bolus amount may be presented to the user, for example, via the touch-sensitive screen;

Finally, the bolus amount is delivered (controlled by the processor). In some embodiments, the delivery may be carried out automatically (i.e., based on the bolus calculator determination without any interference of the user. In some embodiments, the delivery may be carried out "semi-automatically" with some interference of the user, for example, the user has to confirm the delivery or may change the recommended bolus amount at his/her discretion. In some embodiments, the user may reject or ignore the bolus calculator recommendation and to either deliver the drug or not.

The network to which the disclosed injection device can be connected can, for example, be any kind of network, such as Internet, Intranet, Wi-Fi, Bluetooth or a mesh network (ad-hoc network). It can correspond in particular to the network enabling a system of networked medical devices.

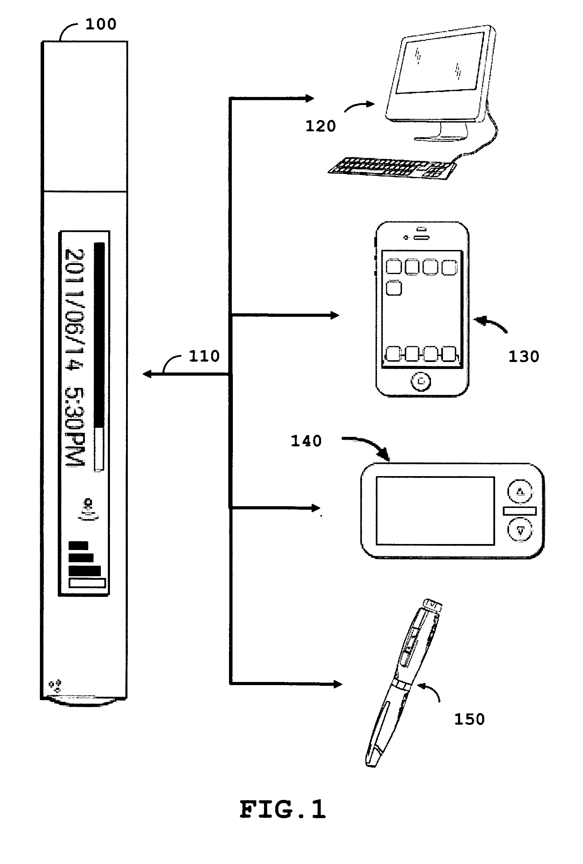

FIG. 1 illustrates how a disclosed injection device (pen or insulin injection device) can be capable of communicating with external devices, either medical devices such as blood glucose monitor/meter (stand alone, IBGM, CGM), dedicated remote control, insulin delivery device (e.g., pump, another pen) and/or other generic devices such as PC, laptop, PDA (dedicated or generic), cellular phone, smartphone, media player (e.g., iPod), TV, and the like. Communication may be configured as RF communication, Bluetooth, IR, and any other means of wireless communication. In some embodiments, the communication may be wired (e.g., USB cable). Communication may one or more of: unidirectional (e.g., injection device to external device, external device to injection device), bi-directional (e.g., injection device to and from external device) and polydirectional (e.g., injection device to and from multiple devices, communication with a network, cloud, etc.)

The figure 1 shows the insulin injection device 100 in interaction through the network 110 with personal computer 120 and/or a mobile phone 130 and/or a tablet PC 140 and/or another insulin injection device (standard insulin injection device or an injection device according to one or more embodiments of the present disclosure). The insulin injection device communicates with networked medical devices and/or IT (information technology) devices. The cooperation of devices enables new interactivity schemes in diabetes management. The display of the insulin injection device 100 can be complemented by a display of the personal computer 120, when passing by, for example. One or more of the medical or IT device also can send commands to the insulin injection device. For example, the smartphone 140 can trigger the request for a bolus to the insulin injection device 100. A doctor can retrieve or update data via the network. All parameters of the insulin injection device, including firmware, operating system and software applications can be modified through the network. Access rights or credentials also can be managed by a central logic (centralized or distributed over surrounding devices).

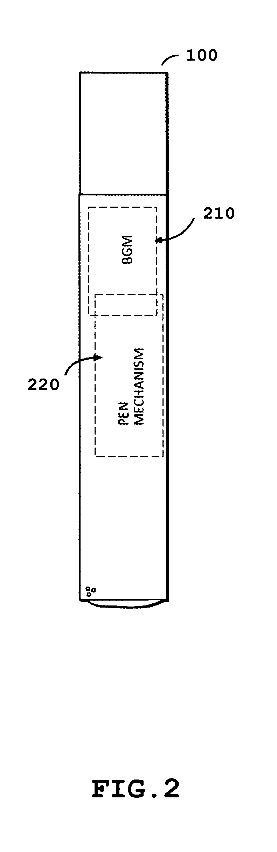

FIG. 2 illustrates an example of an injection device configured as an insulin injection device including both insulin delivery means (i.e., "pen/injection device mechanism") and analyte sensing means, i.e., blood glucose monitoring ("BGM").

The figure 2 shows a feature of the insulin injection device according to the disclosure, in that it integrates a blood glucose meter. The techniques used for measuring the blood glucose levels can be optical or electro-chemical.

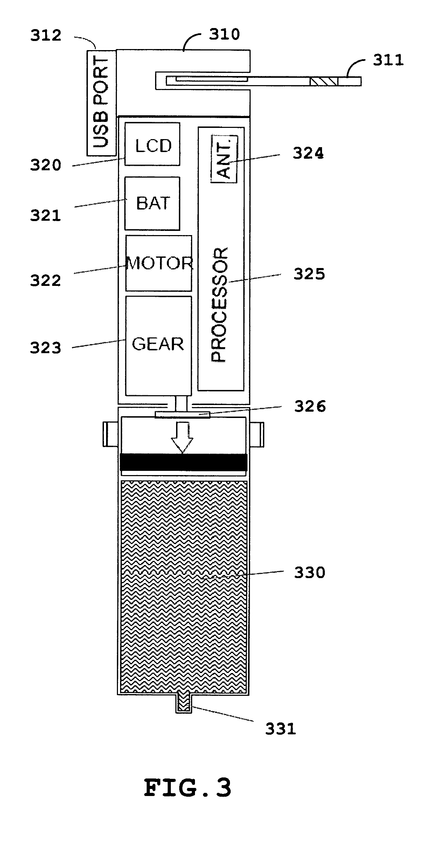

FIG. 3 provides a general description of exemplary components of an embodiment of an insulin pen or injection device. In this instance it comprises:
- a reservoir 330 for retaining the drug (e.g., insulin). The reservoir can be flexible or rigid. It can be pre-filled or not. It can be disposable or reusable. The reservoir can consist in an extension connectable to the injection device or can consist in a pre-connected part. The reservoir is suitable for insulin storage, but can also be suitable for storing any other drug (for example glucagon).
- a driving mechanism including a motor 322 (e.g., stepper motor, DC motor, piezoelectric motor or any other type of driving mechanism), one or more gears 323 (e.g., planetary gear), and a plunger/piston 326 for displacing the drug from the reservoir to the body of the patient. The driving mechanism can also be constituted by micro mechanisms, such as MEMS (micro-electro-mechanical systems). According to such a micrometric design, a volumetric membrane comes with a pair of check valves integrated in MEMS chip. The chip is a stack of several layers bonded together: a Silicon-On-Insulator SOI plate with micromachined pump structures and two Pyrex cover plates with through holes. The MEMS chip is assembled with a piezoelectric actuator that moves the membrane in reciprocating movement to compress and decompress the fluid in the pumping chamber.
- a power source (e.g., one or more batteries, one or more capacitors, or a micro fuel cell) for providing power at least to the driving mechanism. The battery can be rechargeable or replaceable /disposable. According to another embodiment, the source of energy comes from a dynamo and/or a capacitor for storing the energy. According to yet another embodiment, the source of energy comes from energized spring(s).
- a processor/controller 325. The CPU 325 can be one physical entity, but it can designate a unit which controls and/or retrieves and/or distributes computing tasks to other electronic components or via or to the local or remote network (cloud computing). The element 325 also designate memory means.
- communication means 324 (e.g., antenna, transceiver) for communicating (wirelessly) data and/or commands with networked medical / IT devices. In one embodiment, said communication means replaces the processor 325 (computation of the data is not performed locally). In other embodiments the communication means is part of the processor/controller unit
- data transferring means 312 (e.g., USB port, memory card slot) for transferring data (e.g., logbooks including for example data related to infusion and/or data related to BG monitoring) to and from the injection device. The injection device can optionally comprise such ports.
- input means (not shown) for entering data to the injection device and controlling at least the injection device such as one or more of: a touch-sensitive screen, key(s), keyboard, button(s), audio commands, camera, etc.
- output means 320 to notify the user and provide indications (e.g., visual, audible, vibrational) such as one or more of: a touch-sensitive screen, microphone, buzzer, vibrator, pico projector, braille screen, etc.
- analyte monitoring port 310 such as a port for receiving a BG (blood glucose) strips 311. The present insulin injection device comprises a BGM (blood glucose meter).

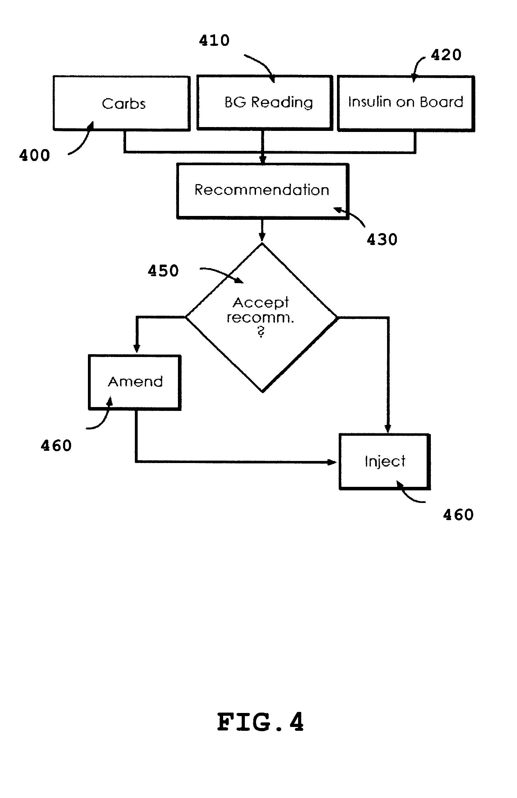

FIG. 4 shows the general usability of an injection device according to the disclosure, and in particular the associated method of using the injection device. At step 400, a value of carbohydrates is received. At step 410, a blood glucose value is received from the BGM of the insulin injection device. With these two values, the embedded bolus calculator calculates a bolus recommendation 430. The patient at step 450 either accepts and injects 460 said bolus or amends 460 and injects the bolus.

Referring to steps 410 to 420, the carbs estimation may be received via the input means (e.g., touch-sensitive screen) and may be associated with a database stored in a memory (e.g., food database, images of foods, caloric tables) and/or with a bolus calculator. The BG value(s) may be inputted via the input means, received from a remote sensor and/or received from a BG meter such as the one coupled to the BG strip port located on the injection device. The Insulin-On-Board may be inputted via input means, calculated by the processor or retrieved from a memory. Referring to step 430, the determination of the bolus amount may be carried out in various ways (local glycemic model or accessed remote model, or doctor advice for example). Referring to step 450, presenting at least the bolus amount can be implemented by the output means (e.g., touch-sensitive screen). Referring to step 460, the amendment can be implemented via input means (either at the injection device or at an external device being in communication with the injection device). Referring to step 450, if the user selects "Accept", then the injection device may deliver the recommended bolus amount (either immediately or with a delay configurable by the user). If the user selects "Edit", then the user may change the recommended bolus amount and only then would the injection device deliver this amount accordingly. If the user selects "Reject", then the process of delivery is cancelled.

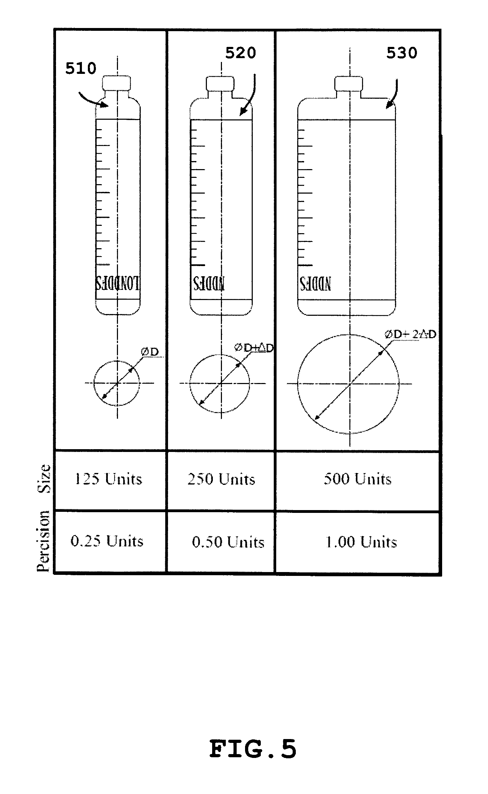

FIG. 5 illustrates various sizes of reservoirs or cartridges. Each of them is characterized by different dimensions (e.g., radius). The injection device is configured to receive the different cartridges (retaining different amount of drug). This is being enabled due to the fact that all cartridges include the identical distal ends (including the cartridge outlet) and identical proximal ends (including the interface with the plunger rod. In the first example 510, the size is 125 units (precision 0,25 unit). In the second example 520, the size is 250 units (precision 0,5 unit). In the third example 530, the size is 500 units (precision 1 unit).

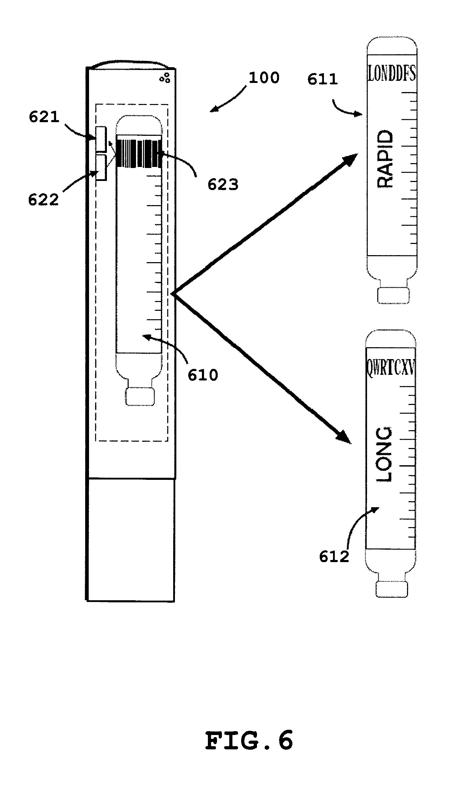

FIG. 6 illustrates an implementation of a recognition/identification mechanism which enables the identification of a cartridge and its characterization, for example, the type of insulin (rapid or long; different types of rapid (e.g., Lispro, Aspart) retained within the cartridge, insulin expiry date, manufacturer, cartridge batch/lot, etc. The identification mechanism may include for example a barcode. In such an embodiment, the injection device includes a barcode reader 621/622 and the cartridge includes a corresponding barcode ID 623. In case where a non-valid cartridge is inserted into the injection device, the processor may reject it and not allow its usage.

Other identification mechanisms may include color detection, RFID, holograms, OCR, etc. Encryption and authentication mechanisms can be added.

An advantage of the present embodiment if that the consumption of insulin can be monitored (possibly in real-time). For example, insulin manufacturers can aggregate data from insulin injection devices through the network and adjust production or financial forecasts accordingly. Statistical analysis can provide useful data (detection of leaks, abnormal low or high consumptions, singular events for example), which in turn can lead to valuable remediation (alerts by SMS or messaging systems, firmware updates, etc). Patients can benefit downstream from automatic repurchasing options (when the reservoir level falls under a predefined threshold, an order for a new cartridge is prepared and/or sent). Compatibilities between insulin cartridges and insulin injection devices can be managed (the behavior of the insulin injection device can be driven by the detected cartridge type for example). Administration and dosage also can be defined according to the cartridge ID. Authentication options also can be implemented (ID codes can guarantee the quality of the drug), as well as other parameters (tracking options such as temperature history, validity date). The payment (in totality or in part) can occur at the first insertion for example, according to a particular business model. Various reporting options are enabled by this "intelligent" identification.

In yet another embodiment, the software implemented for the management of the disease is defined by the identification of the drug being inserted (for example, if an U500 insulin cartridge is detected, the treatment will be different compared to a U100 insulin type). Injection algorithms, bolus calculation, glycemic model, bolus patterns, as well as the user interactivity model can be modified.

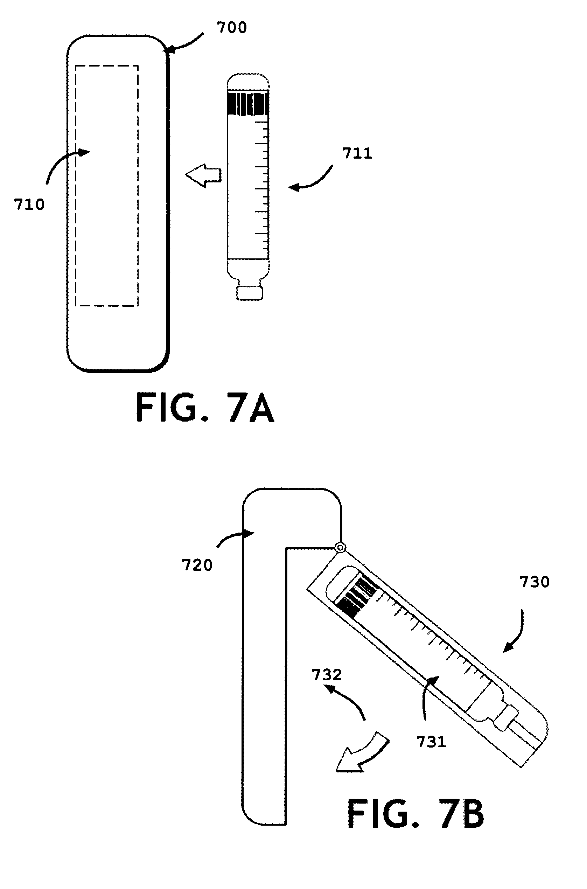

FIGs. 7A to 7B illustrate various configurations of the insertion/loading of the cartridge into the injection device. For example, as shown in FIG. 7A, the cartridge 711 may be loaded into a lateral opening/window 710 of the injection device. FIG. 7B shows another embodiment in which the cartridge 731 may be loaded into a hinged "barrel" 730. In another embodiment (not shown), the cartridge can be loaded from a bottom opening of the injection device housing. Unlike conventional loading opening (e.g., lateral), the bottom opening enables a smaller dimension of the device (no need for an additional lateral opening, additional cover for this lateral opening), and reduction of manufacturing cost (COGS).

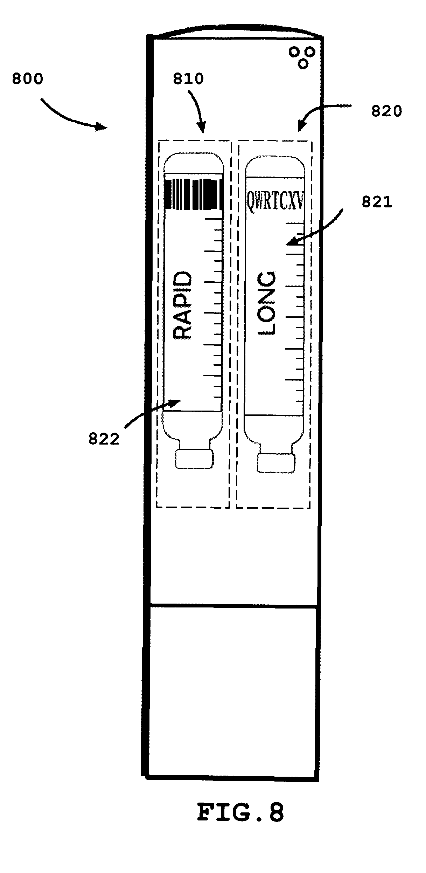

FIG. 8 shows a dual-cartridge embodiment. The injection device 800 can include a plurality of cartridges, for example two cartridges: one cartridge retains long acting insulin 821 and another cartridge retains rapid acting insulin 822. In such an embodiment, a plurality of barcode readers 810 and 820 can be used.

The management of diabetes can leverage the presence of these two types of insulin (a bolus for a food intake with rapid insulin and basal injections with long acting insulin for example). Much more complicated schemes can be implemented, in order to adapt to the physiological response of the patient to the injections. In particular, the existence of a continuous blood monitoring implemented on the patient can enable an optimized glycemic control (feedback loops, real-time adjustments, machine learning algorithms, flexible rapid/long injection patterns, individualized values, etc).

As a generalization, the drug injection device can comprise a plurality of cartridges (2 or more), corresponding to different drugs. Patient treatments can be optimized and individualized (mixing rapid and long acting insulin for example). Several diseases can be treated at the same time.

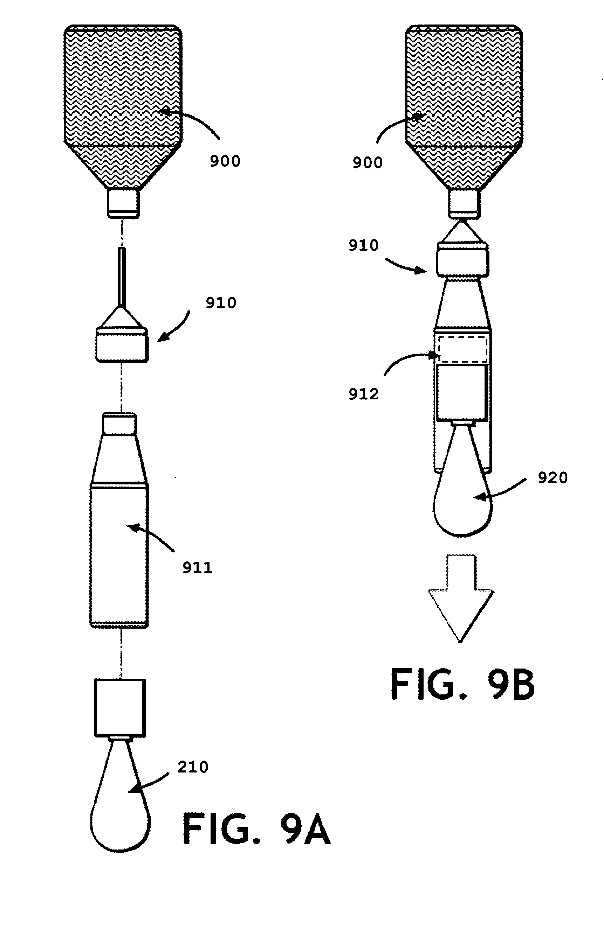

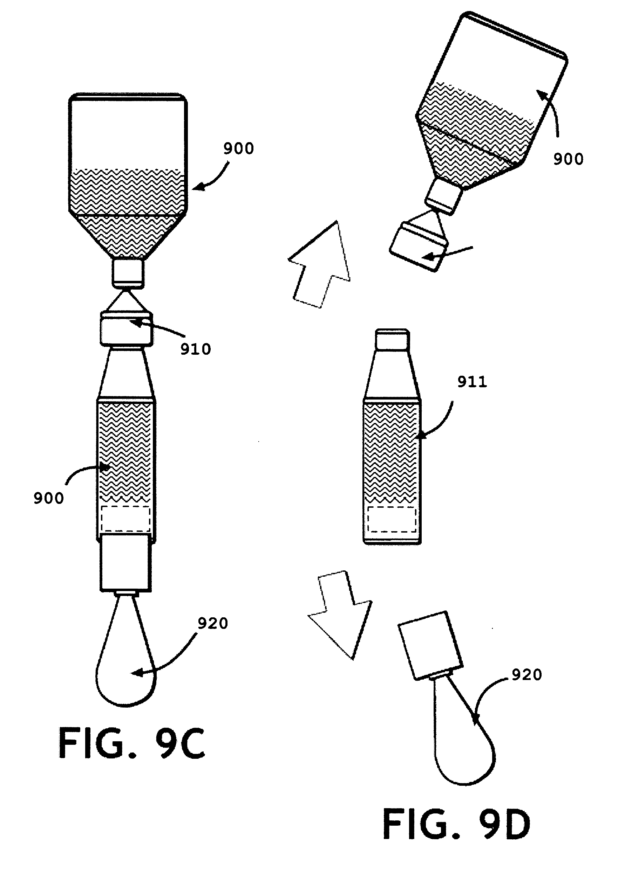

FIGs. 9A to 9D illustrate to the filling process of a cartridge 911. Such a cartridge may be formed of plastic (e.g., polypropylene) or glass and can be disposable or reusable. To transfer the drug from a vial 900 to the cartridge 911, an adapter 910 (which can be disposable) may be used. The adapter couples to the vial on the adapter's distal end and to the cartridge on the adapter's proximal end. Then a handle 920 (which can be, for example, reusable) may be coupled to the cartridge (at the cartridges proximal end). Thus, a user may fill the cartridge at his/her discretion based on their personal use and experience - one user may fill in X units of insulin and another user may choose to fill in Y units of insulin (see FIG. 9c). After filling the desired amount, the adapter and/or handle may be disposed. In another embodiment, a pre-filled cartridge may be used and loaded into the injection device.

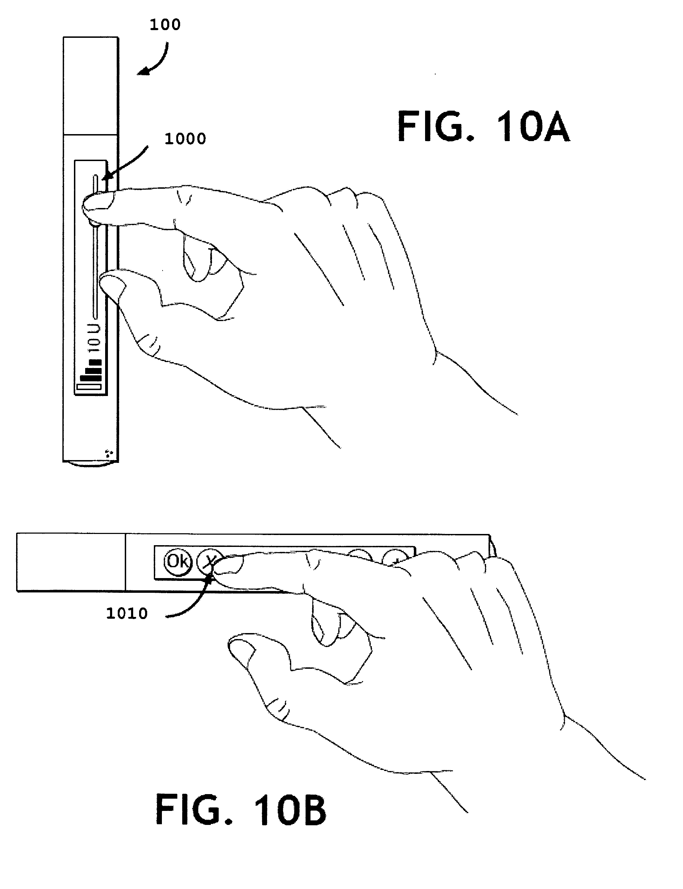

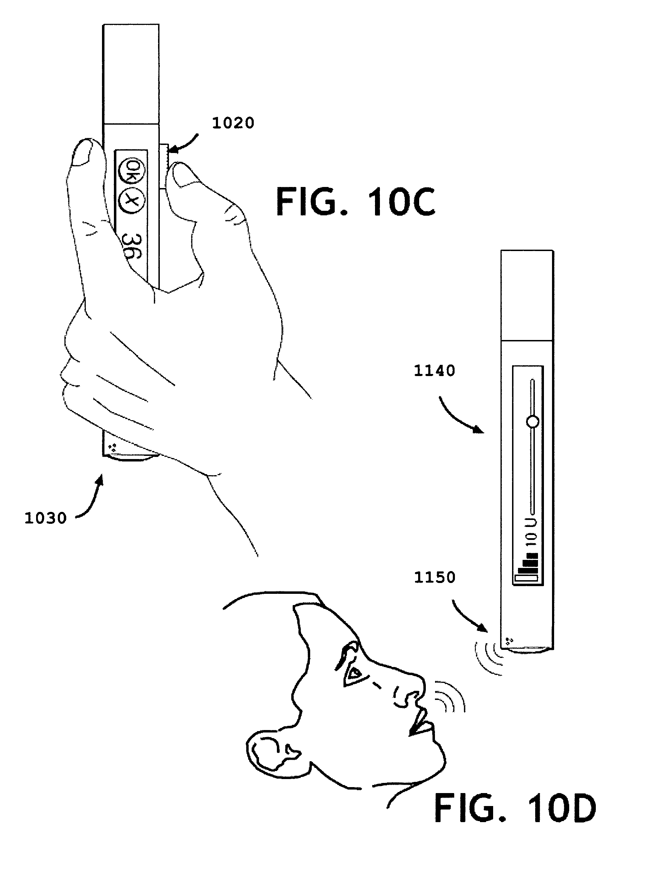

FIGs. 10A to 10D illustrate various input means located at the housing of the injection device 100. These input means may include a touch sensitive screen and/or key(s). Some embodiments may include a touch-sensitive screen on a first side of the housing of the injection device and a dedicated button on a second side, such as a bolus button. A slidable cursor 1000 (physical embodiment as a slider button such as a resistive slider or software embodiment with the touch sensitive surface) enables the user to select the desired amount of drug to be injected. User interface options 1010 enable the user to confirm, modify or cancel the bolus. The used of a slidable cursor to set the dose to be delivered by the injection device improves discreetness, and combined with display and confirmation of the set bolus, improves safety by ensuring that the amount injected is what is intended by the user.

FIG 10C to 10D illustrate the implementation of voice recognition means on the injection device to input commands/data. The injection device can include a microphone 1030 for receiving voice instructions. This assists impaired/disabled patients. In one embodiment, the patient presses a button 1020 which triggers the activation of the microphone 1030. In another embodiment, an audio threshold level is predefined (audio signals above said threshold trigger the voice recognition system). In other embodiments, the injection device can include a speaker 1050 for audible indications. This type of indication may assist for example, visually impaired patients. In yet other embodiments, the insulin injection device or injection device also includes a camera adapted to interpret gestures, as input means. In other embodiments, a voice synthesizer can repeat what was provided by the user to ensure that the correct input has been received by the device.

The injection device can be configured to enable the user to control the delivery profile ("normal" bolus, but also bolus according to an adjusted profile including a plurality of delivery rates (e.g., equivalent to a "Duo" bolus pattern).

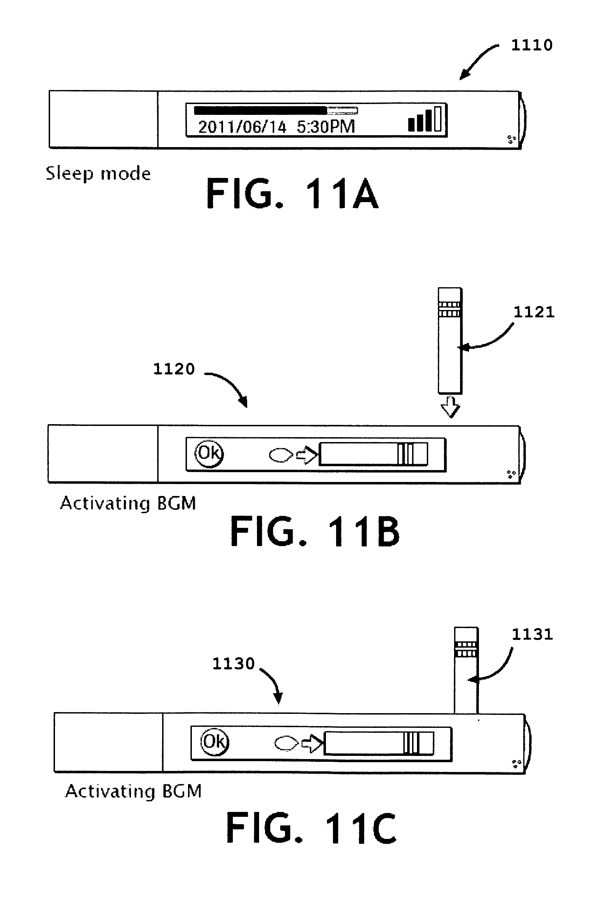

FIG. 11A to 11G show an example of a use of the disclosed insulin injection device.

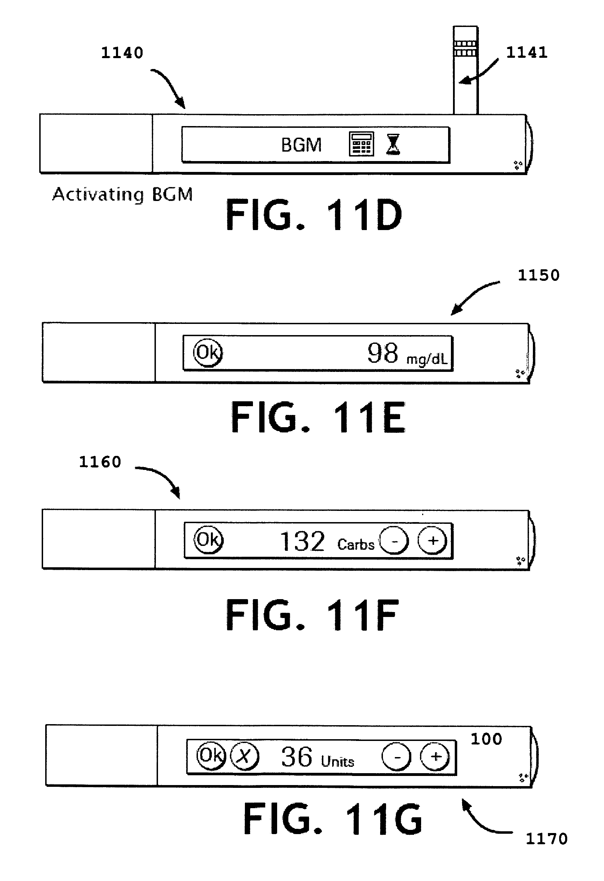

When not in use, the device 1110 may operate in a sleep mode for conserving energy (FIG. 11a). The device is activated, for example, by a dedicated button/switch (or a predefined action performed at the touch-sensitive screen, by a remotely controlled command, and/or by inserting a strip into the BGM port (FIG. 11b-c). Then, the BGM reading can be processed (FIG. 11d), the BG reading may be presented to the user (FIG. 11e), carbs estimation may be inputted or estimated and then presented (FIG. 11f), and lastly, the bolus amount recommendation can be presented to the user (FIG. 11g).

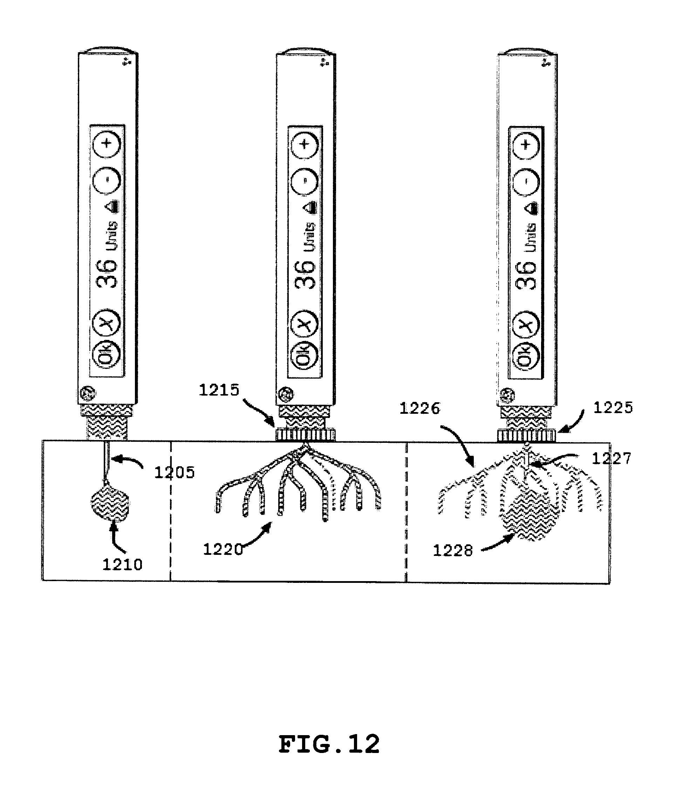

FIG. 12 illustrates an injection device with a particular injection means. In some embodiments, the injection means is configured as an injection needle 1205 including a single outlet which generates a single subcutaneous drug depot 1210. In another embodiment, the injection means 1215 includes a plurality of pores and/or micro-needles which generate a plurality of depots 1220 (transcutaneously and/or subcutaneously). In yet another embodiment, a combination thereof 1225 is implemented (1228/1210 and 1205/1227).

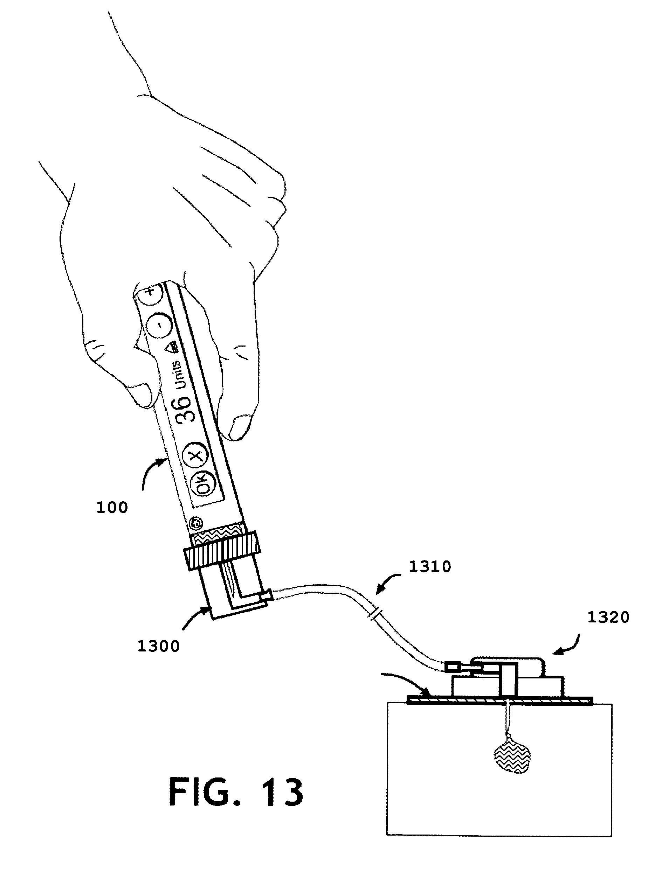

FIG. 13 shows the injection device coupled to an infusion set (either directly of via an adapter). The insulin injection device 100 receives an adapter 1300 connected via a tubing 1310 to an infusion set 1320. This allows the device to be used as a temporary replacement for an insulin pump, if, for example, the batteries or reservoir of their insulin pump are exhausted.

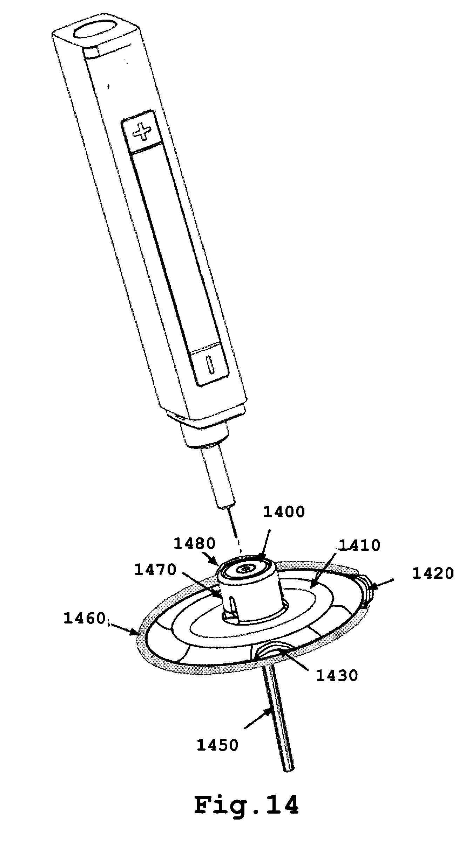

FIG. 14 shows the insulin injection device coupled directly with an infusion port 1400 (disconnection means 1420 and 1430, inlet 1400, needle 1450, adhesive layer 1460, plastic cover 1410).

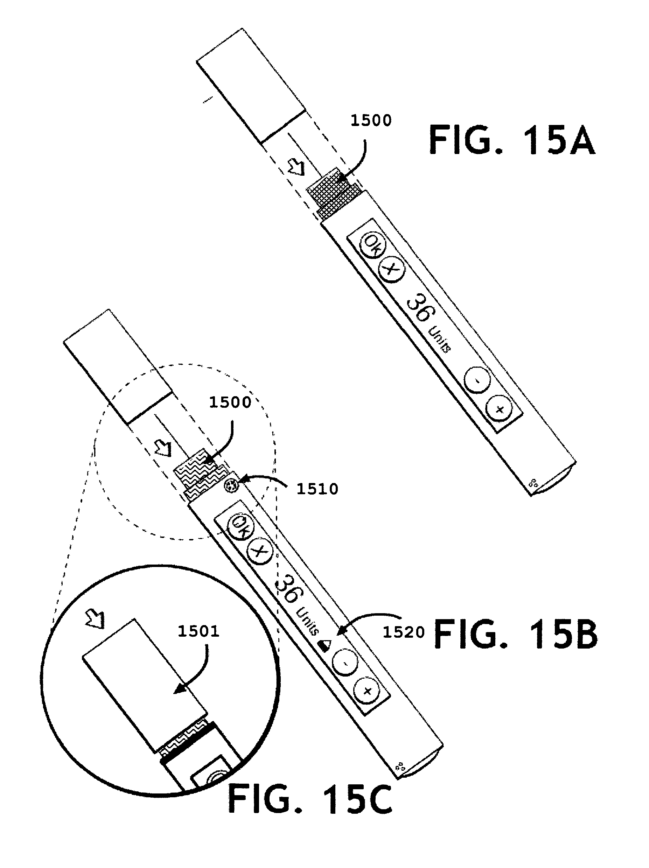

FIGs. 15A to 15C show the insulin injection device configured to enable the user to observe the cartridge and to have a visual indication with regards to the amount retained within the cartridge. This may be carried out via a transparent portion 1500 comprised in the housing of the injection device or via a gap (e.g., slot, slit) between the injection cap (or cover) and body portion, i.e., the housing.

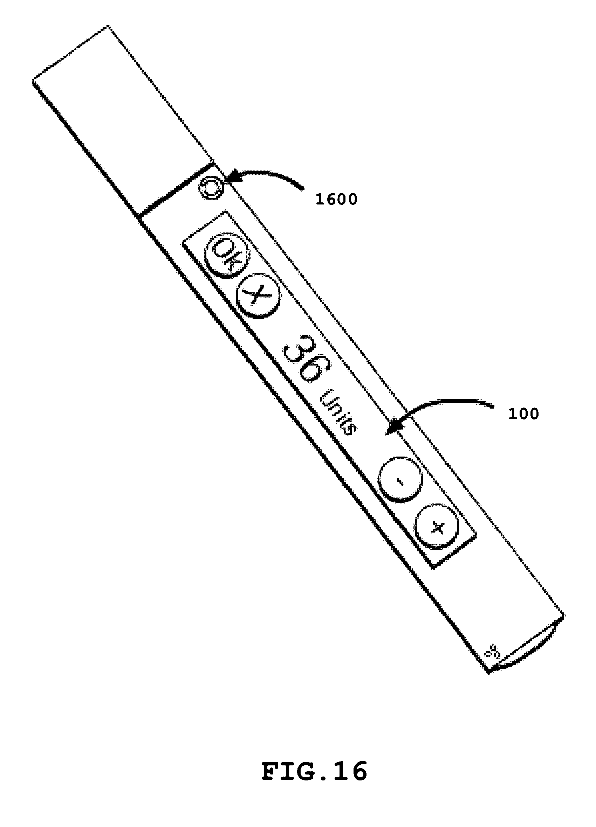

FIG. 16 shows an indicator 1600 notifying the patient whether the cap is properly connected with the housing or not. Such an indication may be configured as a LED operating in conjunction with an electronic sensor which is enabled to determine whether the cap is connected (properly) or disconnected to/from the housing.

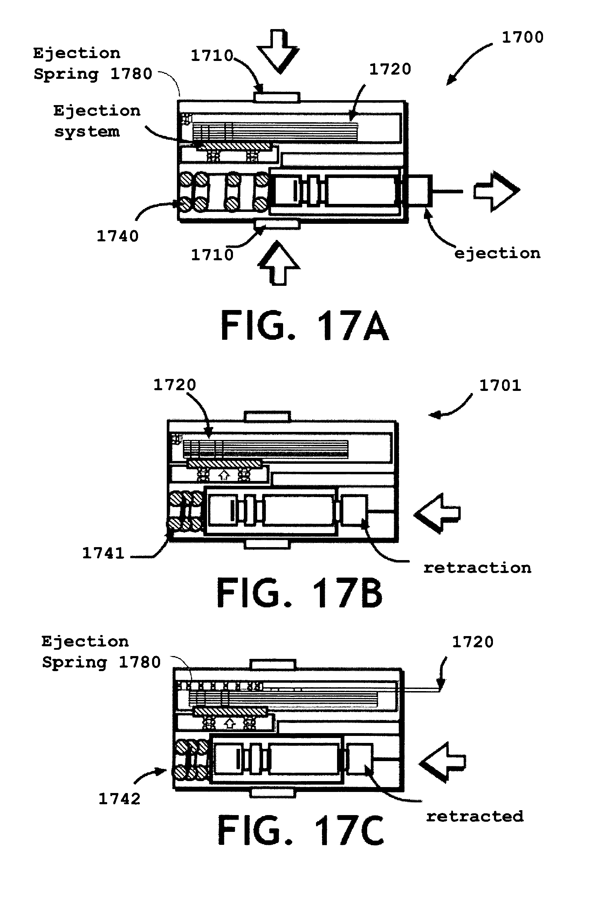

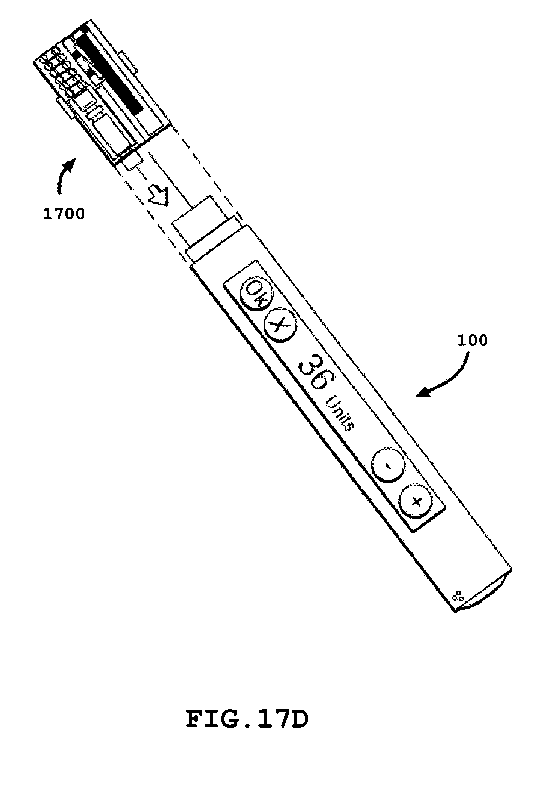

FIG. 17 illustrates an injection device including a cap for at least protecting the injection needle. The cap is further configured to accommodate one or more of a lancing device, BG strips, injection needle, and other elements or devices to be used by a diabetic patient. The user presses the button 1710 and this triggers the release of the needle 1750 (FIG 17A) by the release of the spring 1040. In FIG. 17B the needle is then retracted and this energizes the spring again 1741 while a test strip is partly ejected (FIG. 17B) due to the release of an previously energized strip test ejection spring 1780.

FIG. 17D shows the insulin injection device 100 provided with its cap 1700. Appropriate holes or spaces are placed at the distal end of the insulin injection device and in the cap in order to accommodate both the needle of the insulin injection device and the lancing needle of the cap.

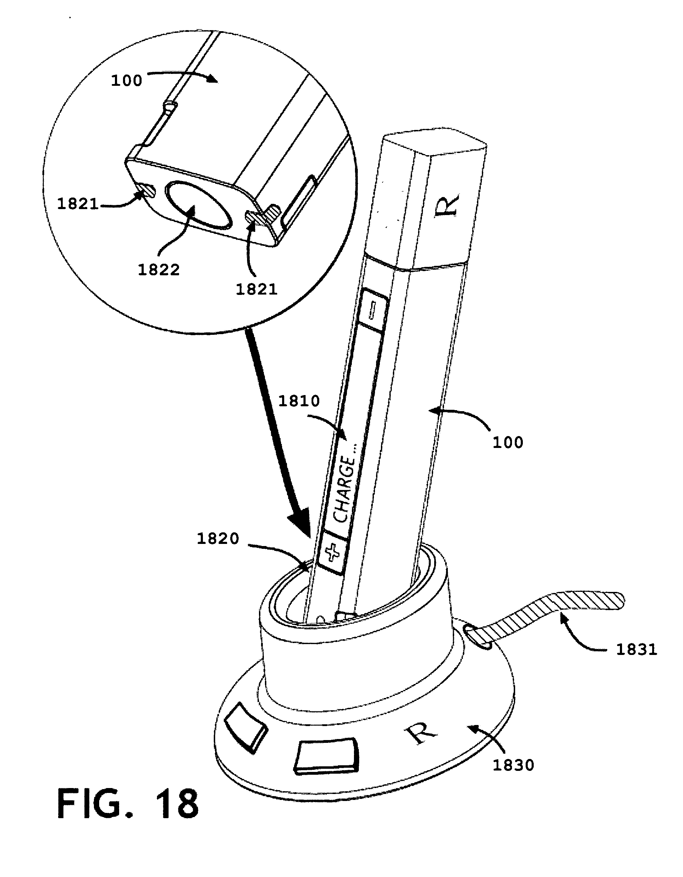

FIG. 18 illustrates the base station 1830 for receiving the insulin injection device 100. In one embodiment, the battery of the pen is rechargeable. When the insulin pen is inserted in the base station 1830 by the opening 1820, the battery is recharged by contact with the electrical contacts 1821. A data transfer can occur by the same electrical components 1821 (all via the communication port 1822). The insulin pen displays the status of the transfer 1810. The base station thus performs a dual function: energy and data transfer. Optionally the base station can be adapted for automatic refueling of insulin (in this case the entry 1822 enables the refilling). The cable 1831, according to the different embodiments, can transmit electricity or insulin or data and or a combination thereof (combinations of tubes and parts). The charging connection may be configured as a wired connection (e.g., USB cable) or a wirelessly connection (e.g., induced charging).

In one embodiment, the base station is connected to the network. If the insulin pen is not provided with communication means, the intermittent connection to the base station enables a corresponding intermittent connection to the network (advantages and effects of such a connection have been discussed in particular in figure 6)

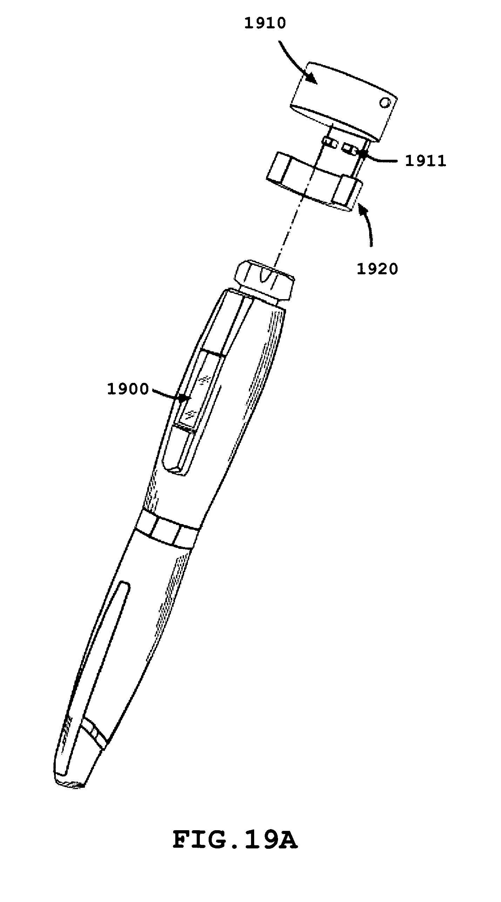

FIG. 19A shows a special communication adapter 1910 for enhancing a standard insulin pen 1900. The adapter acts as a transmitting device which enables to read data from a conventional injection device. The adapter comprises a sensor 1911 and a communication means 1912. The sensor assesses the type and quantity of insulin in the conventional pen and following the results or transmitted via communication means.

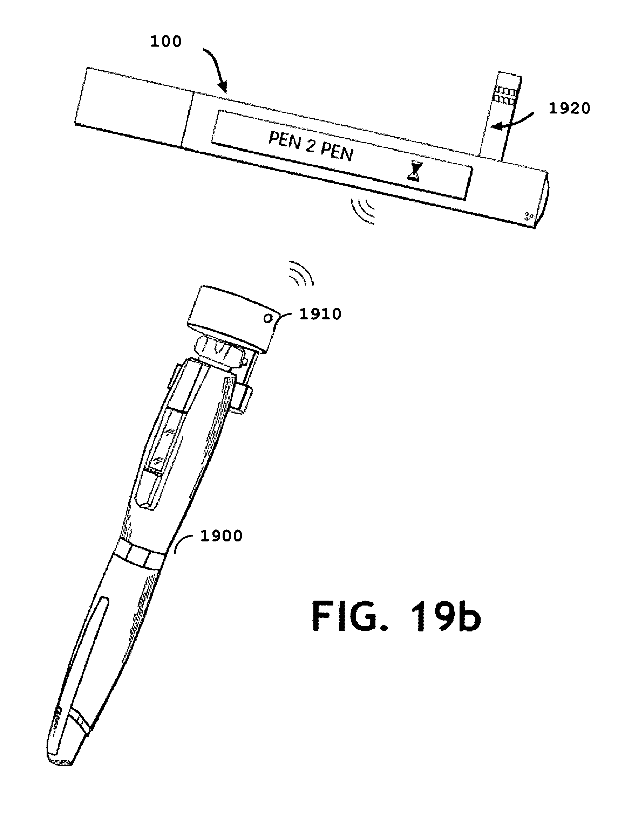

FIG. 19B shows the cooperation of a first injection device, namely an "intelligent" insulin injection device 100 (according to the present disclosure) with a second injection device, namely a standard or conventional insulin pen 1900 provided with said communication adapter 1910.

When coupled to the first "intelligent" injection device, the transmitting device is capable of monitoring and recording the amount of insulin injected and/or delivered by the second (conventional) injection device. The corresponding data is stored in the memory of the "intelligent" device (the insulin pen or injection device according to embodiments of the present disclosure). The processing of data is performed by the first injection device.

Various scenarios become possible. One pen can contain rapid acting insulin while the other one would contain long acting insulin. The communication of data enables an optimization of bolus calculations. The record of injections by one pen enables optimized subsequent injections by the other pen.

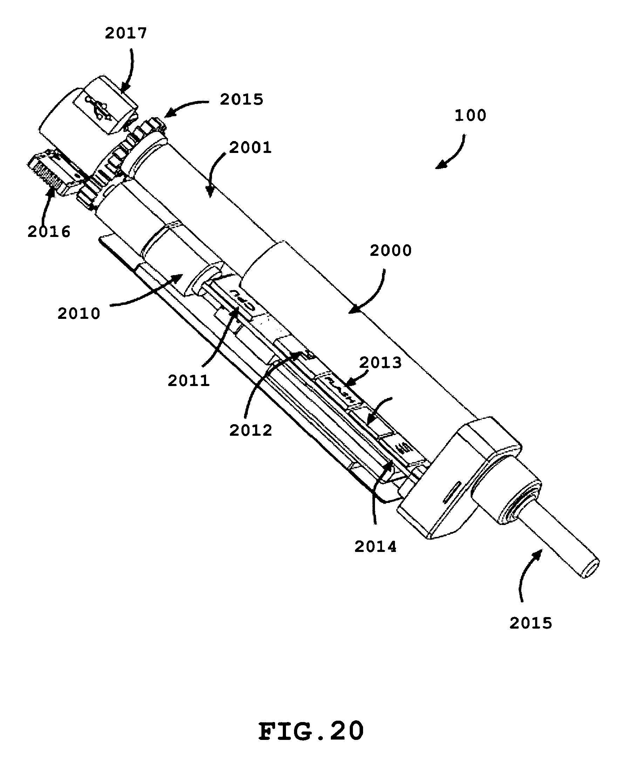

FIG. 20 shows a view of the insulin injection device according to some embodiments of the present disclosure. The drug injection device 100 comprises a reservoir 2000 (for example containing insulin) with a plunger or piston 2001, activated by gears 2015 connected to a motor 2010. The drug injection device comprises an analyte meter 2016, for example a blood glucose meter. The insulin injection device further comprises a USB port 2017, a CPU 2011, a memory 2013, a communication unit 2014, a controller or bolus calculator 2012, the needle 2015 (retractable or not, covered or not). Many different configurations of the drug injection device are possible. For example, the CPU or computing means, the memory means, the controller means can be remotely accessed via a network, instead of presenting local and physical implementation.

Further options and details include: a telescopic screw mechanism, USB connectors and isolators, a Li-Ion Polymer Battery, bolus button switch, BGM Connector, encoder, buzzer, cartridge locking mechanism, cartridge identification.

As fluid delivery or drive mechanism, the further embodiments are proposed:
- Hydraulic Pump (the hydraulic pressure pushes the cartridge's plunger)
- Ball Chain (the lifter Motor lifts one ball every time, a special fork takes each time one ball upwards)
- Micro Piston Cyclic Pump (one-way valves keeps the insulin going only to the outer needle, requires disposable parts)
- Internal "cart" (direct rolling of wheels on the inner part of the cartridge)
- Drums Propeller (3 angled rollers move in a thread-like spiral inside the cartridge)
- Telescopic "antenna" (a cable pushes the plunger, a telescopic "antenna" supports the cable)
- Magnetic Coil (a magnet pushes the plunger, magnetic field comes from a coil outside the cartridge)
- Popping Nut (after half travel, the motor goes back, then the outer nut part stays in place, while the inner part extends)
- Telescopic Screw/Nut

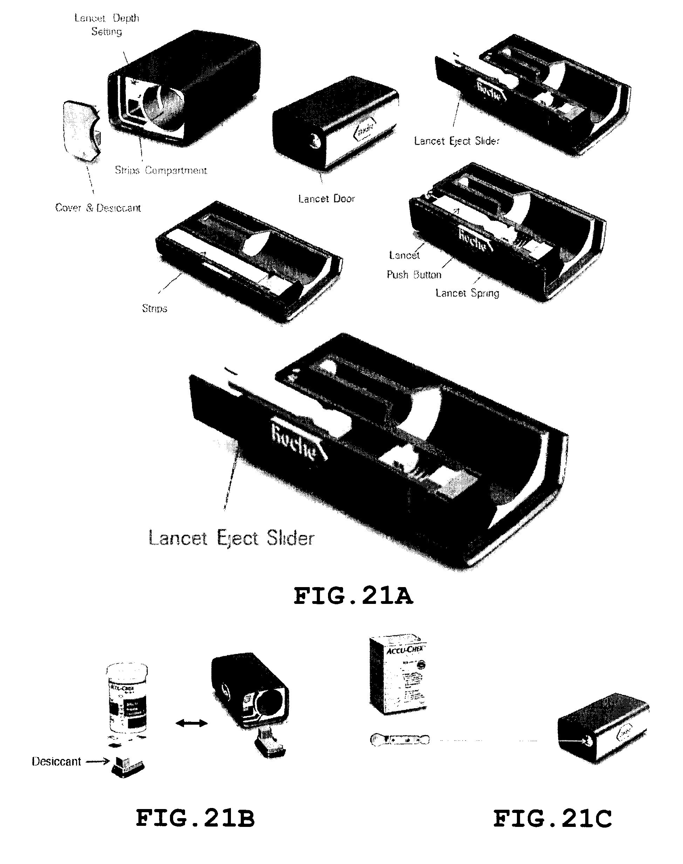

Figure 21A to 21C show the cap comprising lancing elements and test strips, as well as a strip compartment cover with desiccant.

As shown in figure 21A, the cap comprises the lancing elements arranged in a compact form. It comprises: a lancet depth setting mechanism (in this case a set screw) that permits a user to adjust the depth of lancet penetration to personal preferences and/or needs, a strip compartment for holding measurement strips such as blood glucose measurement strips, a lancet door (for insertion of disposable lancets), a lancet eject slider (actuated by the user) that can also function as a drive mechanism tensioner, a lancet (disposable or reusable in some embodiments or with a plurality of lancets), a push button (pressed by the user), a lancet spring (one or more for automatic insertion and retraction of lancets), (disposable) strips, a cover including a desiccant compartment for holding a desiccant (the cover can be disposable and/or the desiccant can be renewable such that it is changed periodically to keep the humidity within the strip compartment low; for example, a new cover can be provided to a user in a container along with a certain number of measurement strips), one or more springs (for ejection of strips) The slider, when moved, energizes a spring. The user can adjust the depth setting. When the push button is pressed, the spring is released and the lancet is ejected to the preset depth.

According to a more particular embodiment, a container will contain test strips and one or more strip compartment covers including a desiccant within or one or more desiccant modules that can be replaced in the cap. The user will thus be able to refill the cap of his/her insulin pen with new disposable test strips and one or more renewed desiccant caps. The purpose is to get a subset of the previously available disposable elements (preserving and optimizing portability) and maintaining the test strips in an environment that helps ensure their accuracy and precision over repeated measurements.

As shown in figure 21B, the cap is provided with a compartment for strips (which must not be wet or humid). The compartment comprises an opening (or a window or an access or an entry or a hole). Said opening (or equivalent) is covered by an element (piece of plastic, of metal, of wood, or resin, of polymer) covered by desiccant. As shown in the figure, the element covered by desiccant fits into the opening of the compartment. This arrangement helps makes sure that test strips are maintained in a low humidity environment. A desiccant is a hygroscopic substance that induces or sustains a state of dryness (desiccation) in its local vicinity in a moderately well-sealed container. Desiccants are often solids, and work through absorption or adsorption of water, or a combination of the two. Other desiccant may work through other principles, such as chemical bonding of water molecules. Desiccants remove excessive humidity that would normally degrade or even destroy products sensitive to moisture. Some commonly used desiccants are: silica gel, activated charcoal, calcium sulfate, calcium chloride, montmorillonite clay, and molecular sieves. Rice or salt are common "low-tech" alternatives.

As shown in figure 21C, the cap also can also house pre-packaged lancets, of common or proprietary format.

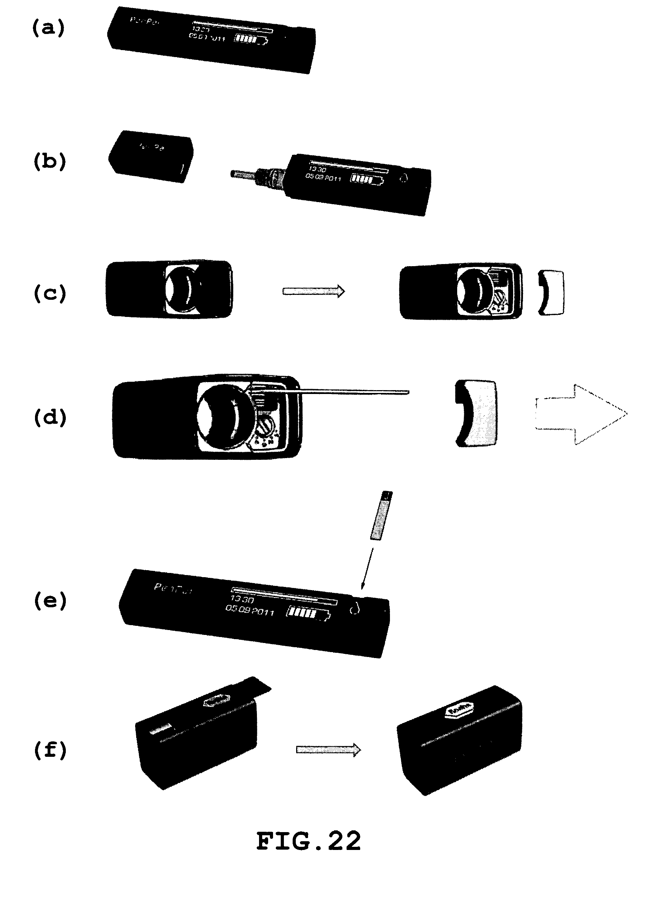

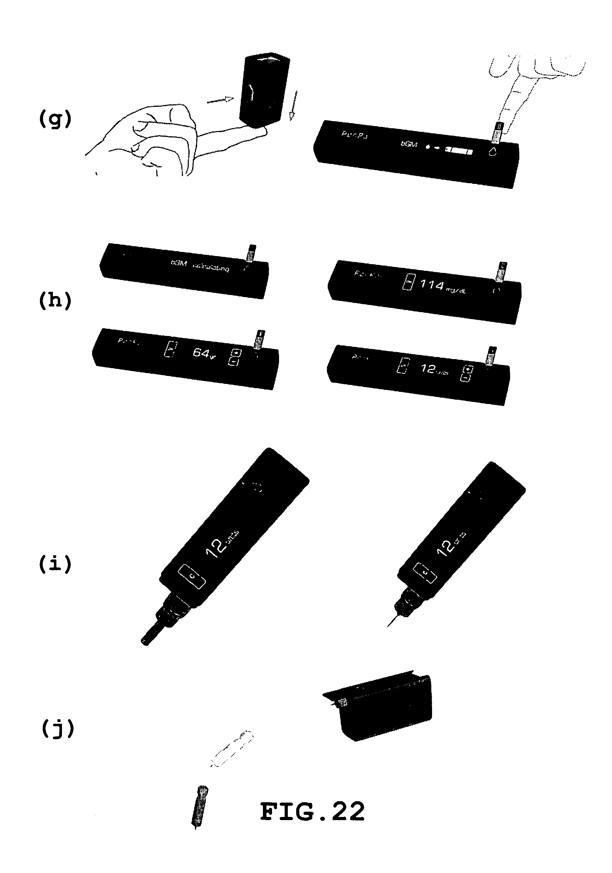

Figure 22 shows steps of an example of the use of the injection device with the cap comprising lancing elements and test strips. At step b) the cap is removed (and the injection device can be powered up if a sensor or connector is present to initiate the start-up sequence when the cap is removed, for example, the user interface can switch on, etc., thereby helping to reduce power consumption while the cap is in place but making it unnecessary for the user to do something other than removing the cap to turn the device on). At step c) the user removes the element (which optionally contains a desiccant as described above) covering the strip compartment. If not done previously as a default during manufacture or previously by the user, the user can access the lancet depth adjustment mechanism once the covering element is removed. The user then extracts manually or automatically a test strip out of the compartment. At step e), the user inserts a test strip into the injection device (in the blood glucose meter for example) At step f), the user slides the lancet eject slider and this energizes the spring contained in the cap. At step g) the user pricks his/her finger and immediately after brings the blood in contact with the test strip previously inserted in the blood glucose meter. At steps h) the BGM calculates then displays the BG value, the user enters a carbohydrate value corresponding to the food intake (consumed or planned in the near future) and the BG displays the recommended bolus. At step i), the needle is uncovered and the bolus is injected. At step j) the disposable lancing element is removed (and replaced)

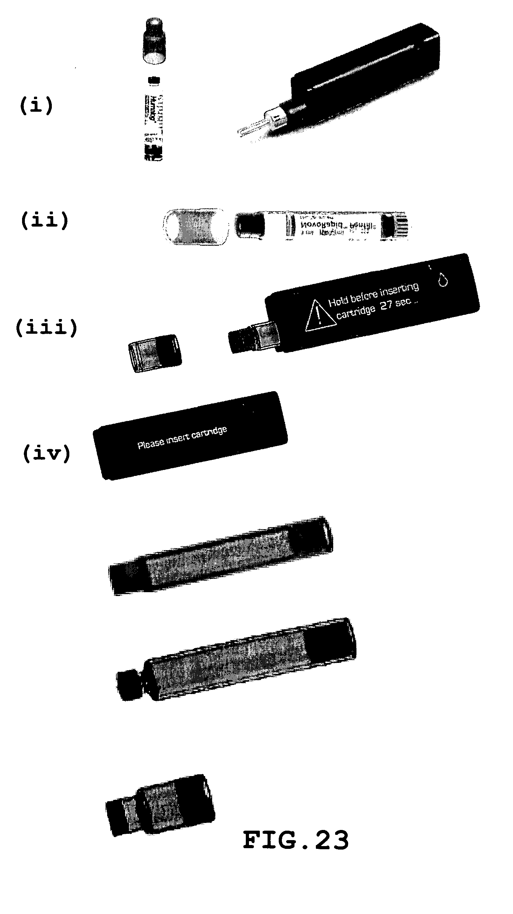

Figure 23 shows different adaptors to accommodate different insulin cartridges. i) shows an adapter for a first format ii) shows an adapter for a second format iii) shows the replacement of a cartridge and iv) shows different cartridges format.

In one particular embodiment (not shown), the injection device presents a pen shape, comprises a BGM, a touch sensitive screen; a physical bolus button, a motor, a bolus calculator and a single housing (not including the cover). A scenario of use is the following: BG level is sensed via BGM (+strip). Result is presented on Screen. Bolus is calculated based on BG reading and other parameters (e.g., ITC, BOB) are stored in memory. A recommended bolus dose is presented on screen. The dose can be adjusted via touch sensitive screen. Initiation of delivery is carried out following activation of physical bolus button. Accurate delivery of insulin is made possible by the motor-based pumping mechanism. Delivery characteristics are stored in memory. All is done within a single housing. As further options: a) BGM is non-invasive (e.g. optical-based) and does not include a strip. Pricking is not required. b) Physical Bolus Button is configured as by voice commands, eye movement or gesture commands. c) Bolus Calculation is based on other physiological parameters (e.g., heart rate, ventilation, blood pressure) which are monitored in real-time. d) touch-sensitive screen presents information in Braille for visual impaired patients. Or no need for screen - information is projected on the retina of the user. Among advantages and effects: the pen has a memory and this implies a better glycemic control because more accurate values are used for calculations; Ease of use (user carries a single compact device); Accurate delivery (motor-based) better glycemic control ; the touch Screen enables compact I/O in one device; the physical button for bolus is provided as a safety ; the device is a self-contained compact device - auxiliary components (e.g., remote control, computer, stand-alone glucometer) are not required; the wired communication between delivery and sensing functions reduces the risk of loss of data; the integration of functions increases the robustness of the device and of the data management. In other words, there is described an integrated system for diabetes (bolus) management; or a clinical integrated I/O system (input is BG level, output is delivery of insulin); or an intelligent bolus doser; or a digitalized controlled insulin pen.

According to another embodiment, the pen is provided with reminder, alert and alarm means. A scenario is a "duo" bolus pattern. A first dose is injected. After a predefined time a reminder prompts for a second bolus dose. Another scenario is an "degraded basal"; the pen is used as a MDI-like device (Multiple Daily Injection) and serves as "backup" for the basal injection (for example one prink per hour may be acceptable in absence of a patch or an insulin pump; alternatively, the user may set the compromise he wants, each 30 mn for a degraded bolus for example) A predefined time may be setup, either (a) fixed (b) variable (b1) defined by a logic in the cloud or (b2) defined by a logic implemented in the local networked devices. The context of the user may be taken into account (the accelerometer can postpone the second bolus by maximum 15 minutes if in a night club for example). Advantages and effects relate to a better system regulation, a better glycemic control. In particular, the first injection may condition the start of the measurement of time and no injection may be possible before said time is elapsed (integration/combination). In other words, the injection device is "intelligent". The disclosed solution can be an enabling technology for a semi-automated structured testing. The solution relates to time management and basal management. It also may constitute an emergency regulation mechanism.

In yet another embodiment (not illustrated), the pen or injection device comprises communications means and alert/alarm/reminder means. This connection to the network enables alerts to be sent to parents via SMS for example. The connection (even intermittent) enables synchronization with cloud hosted calendars for alerts, alerts and reminders. For example, if and when the USB pen is connected to the network the level of insulin can trigger the purchase of additional cartridges. A real time monitoring of insulin consumption is enabled for insulin manufacturers. Connectivity means retroaction or feedback loops. This can lead to better stock management.

The disclosure also encompasses the following alternatives:
An injection device for delivering a fluid, the injection device comprising:
   - a housing
   - a reservoir
   - a drive means for expelling the liquid from the reservoir
   - a processor for controlling the drive means
   - an injection means fluidically coupled to the reservoir and protruding from the housing
   - a user interface with data input and data output means
      characterized in that:
      the injection device comprises a blood glucose meter.

As a first development, the device is further comprising communications means adapted to transmit blood glucose values measured by the blood glucose meter to a networked medical device, such as an insulin pump or a continuous monitoring device, or to a device such as a personal computer, a tablet, a mobile phone, or a television.

There is disclosed an injection device for delivering a drug, the injection device comprising:
- a housing
- an emplacement for a drug cartridge
- a drive means for expelling the liquid from the reservoir
- a processor for controlling the drive means
- an injection means fluidically coupled to the reservoir and protruding from the housing
- a user interface with data input and data output means
   characterized in that:
   the injection device comprises a drug cartridge identification mechanism, said identification mechanism enabling the retrieval of data such as type of insulin, expiry date, manufacturer, or batch or lot.

As a second development, the identification mechanism includes techniques such as bar code, color detection, RFID, hologram, OCR or a combination thereof.

There is disclosed an authentication mechanism resulting in the acceptance or the rejection of a drug cartridge.

There is disclosed communications means, the amount of remaining drug in the cartridge being communicable via a network.

There is disclosed an injection device for delivering a fluid, the injection device comprising:
- a housing
- a reservoir
- a drive means for expelling the liquid from the reservoir
- a processor for controlling the drive means
- an injection means fluidically coupled to the reservoir and protruding from the housing
- a user interface with data input and data output means
   characterized in that:
   the injection device comprises a cap for covering the injection means and the cap is configured to house lancing elements such as a lancet and a test strip.

There is disclosed that the cap further houses one or more springs adapted to expel or retract the lancet.

There is disclosed an injection device for delivering a fluid, the injection device comprising:
- a housing
- a reservoir
- a drive means for expelling the liquid from the reservoir
- a processor for controlling the drive means
- an injection means fluidically coupled to the reservoir and protruding from the housing
- a user interface with data input and data output means
   characterized in that:
   the user interface comprises a microphone and voice recognition means adapted to perform tasks such as receiving voice commands,
   recording comments or assessing audio ambience.

There is disclosed an audio speaker adapted to provide audio feedbacks, such as confirmations, to the user.

There are disclosed voice synthesis means adapted to restitute data such as a blood glucose value or a confirmation of an injection.

The disclosure also encompasses the following devices:
- an injection and measurement device wherein the lancing means and/or the injection means can be actuated by voice commands such as "test" or "prink" or "inject" received by the microphone;
- an injection device wherein the lancing means or the injection means can be actuated by voice commands received by a microphone implemented on one of a networked device, such as a mobile phone, in communication with said injection device;
- an injection and measurement device which, when triggered (voice command, button pressed, gesture), prinks the skin of a patient, withdraws a blood sample, analyses the blood, establishes a drug remediation and immediately after (i.e. without a confirmation by the user but according to predefined agreed injections schemes for example comprising thresholds and maximal amounts of drugs over certain periods of time) injects the drug remediation, using the same withdrawal needle or a second and distinct injection means;
- an injection device provided with network connectivity and which continuously monitors available and connected medical devices in the proximity of the patient, establishes a strategy of injection, and upon opportunity injects one or more drugs (injection of a basal dose while a bolus is injected)
- an injection device provided with communications means which communicates data, comprising parameters such as the remaining volume of insulin in one or more cartridges, or the number of remaining test strips, to one or more networked devices, which devices in turn can order cartridges or test strips for replacement or to perform other related tasks;
- an injection device provided with a plurality of cartridges or reservoirs and with computing means, local or remotely accessed, adapted to manage multiple injection schemes and associated dosage administration;
- an injection device compatible with a standard needle or cannula or a specific cannula such as a sprinkler cannula, the mode of injections being controllable by the user interface or by voice commands
- an injection and/or measurement device provided with communications means which transmits measured diagnostics values to the networked devices present nearby and wherein said devices display one or more of said values;
- an injection and/or measurement device provided with communications means which receives data from other networked devices present nearby and which takes said data into account for the drug administration and/or display said data on said injection and/or measurement device;
- an injection and/or measurement device provided with insertion means for cartridges, such as an opening, window, hole, shield, plug or a combination thereof;
- an injection and/or measurement device provided with a cap covering the injection means and a sensor determining whether the cap covers the injections means (open position) or not (closed position) and wherein the screen or user interface sleeps or hibernates in open position and lights on or activates when in closed position.

The disclosure also encompasses the following devices:
a) an injection device provided with cartridge recognition means;
b) an injection device provided with a plurality of cartridges and/or reservoirs;
c) an injection device provided with voice command means;
d) an injection device provided with a cap containing lancing elements such as lancets and test strips;
e) a networked injection device provided with communications means;
f) an injection device provided with a slider (physical element or software embodiment);
ab) an injection device provided with cartridge recognition means and a plurality of cartridges and/or reservoirs; wrong or adapted insertions can be detected and associated warnings can be raised; a management of remaining drugs is enabled (for example, rapid acting insulin can be substituted to long acting insulin by adapting the number and/or frequency of injections);
ac) an injection device provided with cartridge recognition means and voice command means; voice commands can activate or confirm the loading of the identified insulin;
ad) an injection device provided with cartridge recognition means; with a cap containing lancing elements such as lancets and test strips; the number and type of the lancets and/or test strips can be correlated to the type of drug contained in the identified cartridge;
ae) an injection device provided with cartridge recognition and with communications means; the amount of remaining insulin can be monitored and reported.
af) an injection device provided with cartridge recognition means and with a slider (physical element or software embodiment); the slider, in one position, on demand or a certain time intervals, can represent the amount of remaining insulin (no need for a real visual control with a transparent housing for example); the present control is a virtual one, a representation enabled by the position of the cursor.
bc) an injection device provided with a plurality of cartridges and/or reservoirs and with voice command means; for example, the user can choose by voice command whether he wants to inject rapid or long insulin or both;
bd) an injection device provided with a plurality of cartridges and/or reservoirs and with a cap containing lancing elements such as lancets and test strips; the number and type of the lancets and/or test strips can be correlated to the number and type of drugs contained in the plurality of cartridges and/or reservoirs;
be) an injection device provided with a plurality of cartridges and/or reservoirs and with communications means; the management of cartridges can be controlled by or via the network (order, reimbursement, expiry date management, etc);
cd) an injection device provided with voice command means and with a cap containing lancing elements such as lancets and test strips; the prink can be actuated by a voice command for example;
ce) an injection device provided with voice command means and with communications means; for example, a voice command can be transmitted over the network and partly executed on or by the injection device (injection, calibration, etc) and partly on or by the network (record of the audio comment, logbook, command to another device, etc);
cf) an injection device provided with voice command means and with a slider (physical element or software embodiment of the user interface); for example, after a voice command is received, the slider moves, the user looks at the slider to confirm the amount of insulin to be injected. A bright and big bar as a slider is easy to see and valuable for visually impaired.
de) an injection device provided with a cap containing lancing elements such as lancets and test strips and communications means; the number of remaining lancing elements can be monitored and reported;
abc) an injection device provided with cartridge recognition means, a plurality of cartridges and/or reservoirs and voice command means; wrong or adapted insertions can be detected and associated warnings can be raised; a management of remaining drugs is enabled by voice command (for example, rapid acting insulin can be substituted to long acting insulin by adapting the number and/or frequency of injections);
abe) an injection device provided with cartridge recognition means; with a plurality of cartridges and/or reservoirs; with communications means; the management and monitoring of cartridges is enabled, as well as inappropriate insertions for example;
bcd) an injection device provided with a plurality of cartridges and/or reservoirs; with voice command means; with a cap containing lancing elements such as lancets and test strips; voice commands can control the lancing process and/or the injection process.

The following items and aspects are also disclosed:
- a cartridge stability aspect: the pen is provided with a reading unit to read out information about the stability of a fluid within a cartridge (it comprises an optical unit which measures the transparency of the fluid)
- a cartridge lock: once inserted the removal of the cartridge is detected.
- a test strip vial with a lid and test strips and desiccant packages which can be removed to be inserted into the chamber of the above mentioned cap;
- a lancet device with a hole through which a lancet can be exchanged whereby the size of the hole is automatically reduced by a closure during cocking;
- a lancet device with a slider for cocking the drive unit if the slide is moved in the first direction and ejecting the lancet by moving the slider in another direction;
- a pen with a cap and a lancet device integrated into the cap whereby the lancet can only be cocked if the cap is removed from the pen;
- a cap for protecting an injection needle having two further separated compartments for inserting a lancet and test strip whereby the two compartments can be closed and opened independently from each other;
- a device with a measuring unit for measuring an analyte having a processor for computing a value of the measured analyte and a display for showing the value whereby based on the measured value a bolus is calculated and shown on the display and a user interface allowing the user to change or confirm the shown bolus and an adjusting unit for automatically adjusting a fluid dispensing system in accordance to the changed or confirmed value.

In a particular embodiment, the drug injection device is a connected device, but in another embodiment it can operate as a standalone device (online, off-line and intermediate modes). The drug injection device interacts with other networked medical devices, such as pens or insulin pumps or continuous monitoring sensors. It can also interact with so-called Body Area Network (BAN) sensors or Body Sensor Network (BSN). Body sensors comprise wearable and/or implementable and/or wireless (bio) sensors adapted to assess the physiological state of the user or patient. These sensors can monitor said physiological state continuously or not (on demand or upon action of the user). For example, the following parameters can be monitored: blood glucose, heart rate, body temperature, ECG, EEG, EMG, sweat, blood cholesterol, blood alcohol, coagulation, and estimation of physical exercise or carbs consumption (such as evaluated by accelerometers or pedometers for example). The drug pen or injection device is thus part of an integrated medical system. This means in particular that opportunistic approaches are possible: for example, when visiting a diabetic friend, the bolus or basal doses can be administrated with increased flexibility by using devices of third parties, drug administration doses can be fractioned, etc. The drug administration can be distributed over several - and available - medical devices (the presence of such devices can be continuously monitored in the proximity of the patient).

As examples of the value emerging from the cooperation of networked medical devices, the following examples (situations and actions) are provided: a) according to the user profile, for example a child, do a measure at 11 am (highest risk of hypo) b) with an accelerometer, detect excessive shocks c) with a thermometer, detect excessive or fluctuating temperatures ; combined with a degasing model, prompt the user to check tubing for air bubbles; d) if the reservoir becomes too low, combined with an awareness of calendar/schedule, the emergency of refilling triggers an alert or not (a reservoir low at 10am after breakfast bolus is not as important as a low reservoir at 13pm while lunch occurs) e) with a GPS, assess the life situation (for example travelling, known by GPS data) and take further appropriate actions (in car, in plane, working, sleeping, etc). The cooperation of networked medical devices leads to a better disease management, by taking into account more exogenous parameters (anticipation of life events).

Disclosed is a drug injection device, comprising a drug delivery module and an analyte measurement module, such as a blood glucose meter. The device can be provided with I/O means and can be further adapted to communicate with other networked medical or IT devices. Various options are disclosed: different energy sources, a code reader adapted to identify a previously coded drug cartridge during or after insertion, a plurality of different drug cartridges, a sprinkler needle to optimize injections, an adaptor for connection to an infusion set or to a micro pump, a cap covering the needle and containing lancing elements as well as blood test strips, an accessory base station for energy transfer and/or data exchange. Associated systems and methods are disclosed.

## Claims

1. An injection device for delivering a fluid, the injection device comprising:
- a support for receiving a reservoir or a cartridge
- a drive means for expelling the fluid from the reservoir or cartridge
- an injection means fluidically coupled to the reservoir **characterized in that** the injection device comprises an analyte measurement module.

2. The injection device of claim 1, wherein the delivery of the fluid corresponds to a prick, or is configured to oscillate back and forth along a longitudinal axis as the liquid is dispensed from the fluid injection means.

3. The injection device of claim 1, wherein the analyte measurement module is part of a housing of the injection device.

4. The device of claim 1, wherein the analyte measurement module is a blood glucose meter and the fluid is insulin.

5. The device of any preceding claim, further comprising a user interface including data inputting and outputting means, such as a microphone, a speaker, a touch-sensitive screen, a slidable cursor, a physical button, a camera, or a combination thereof.

6. The device of any preceding claim, further comprising communication means, adapted to communicate with other networked devices, such as an insulin pump, an insulin pen, a continuous monitoring sensor, a personal computer, a mobile phone, a tablet, a television or a combination thereof.

7. The device of any preceding claim, wherein the energy source is one of a dynamo, or a spring, or a rechargeable battery, or a solar cell or a combination thereof.

8. The device of any preceding claim, further comprising a code reader adapted to identify a previously coded drug cartridge.

9. The device of any preceding claim, further comprising a plurality of drug cartridges, prefilled or not, and/or drug reservoirs, such as rapid and long acting insulin.

10. The device of any preceding claim, further comprising an injection needle.

11. The device of claim 10, wherein the needle is a sprinkler needle comprising a plurality of holes for optimizing insulin depots during injection.

12. The device of claims 10 or 11, further comprising an adapter to said needle, said adapter being connectable via a tubing to an infusion set or a patch-pump inlet.

13. The device of claims 10 or 11, further comprising a cap covering said needle, containing lancing elements and one or more blood test strips compatible with the blood glucose meter.

14. The device of any preceding claim, further adapted to be received in a base station, wherein said base station is suitable for energy transfer and/or data exchange.

15. A medical system comprising a pen-shaped medical device according to any one of the preceding claims and further comprising computing means adapted to implement one or more functions of a bolus calculator, the implementation of said computing means being local, for example in a processor, or said computing means being accessed remotely via a network.

16. The medical system of claim 15 comprising a plurality of cooperating pen-shaped medical devices according to any ones of the preceding claims and/or with a standard pen mounted with a communication cap comprising a sensor and communications means.
